(19) Europäisches Patentamt
European Patent Office
Office européen des brevets

(11) Publication number : **0 325 472 B1**

(12) # EUROPEAN PATENT SPECIFICATION

(45) Date of publication of patent specification : **28.04.93 Bulletin 93/17**

(51) Int. Cl.$^5$ : **C12Q 1/37,** C12Q 1/26

(21) Application number : **89300533.0**

(22) Date of filing : **20.01.89**

(54) **Composition for testing periodontal diseases.**

The file contains technical information submitted after the application was filed and not included in this specification

(30) Priority : **20.01.88 JP 10241/88**
**28.12.88 JP 331988/88**

(43) Date of publication of application : **26.07.89 Bulletin 89/30**

(45) Publication of the grant of the patent : **28.04.93 Bulletin 93/17**

(84) Designated Contracting States : **AT BE CH DE ES FR GB GR IT LI LU NL SE**

(56) References cited :
**EP-A- 0 252 747**
**EP-A- 0 255 341**
**EP-A- 0 304 871**
**BIOLOGICAL ABSTRACTS, vol. 69, no. 3, abstract no. 64154, Philadelphia, PA, US; D.B. KOHN et al.: "A peptidyl derivative of tritium-labeled aniline as a sensitive, stable, protease substrate"**

(73) Proprietor : **SUNSTAR KABUSHIKI KAISHA**
**3-1, Asahi-machi**
**Takatsuki-shi Osaka-fu (JP)**
Proprietor : **KYOWA MEDEX CO. LTD.**
**No. 6-1, Ote-machi 1-chome Chiyoda-ku**
**Tokyo-to (JP)**

(72) Inventor : **Suido, Hirohisa**
**No. 20-33, Hon-machi**
**Kawachinagano-chi Osaka-fu (JP)**
Inventor : **Miike, Akira**
**No. 410-1, Nameri Nagaizumi-cho**
**Sunto-gun Shizuoka-ken (JP)**
Inventor : **Hasegawa, Kenji**
**No. 11-203, Tamagawa 2-chome**
**Takatsuki-shi Osaka-fu (JP)**
Inventor : **Kayahara, Norihiko**
**No. 2625, Ozenji Asao-ku**
**Kawasaki-shi Kanagawa-ken (JP)**
Inventor : **Eguchi, Toru**
**45-2-508, Tsunoe-cho 1-chome**
**Takatsuki-shi Osaka-fu (JP)**
Inventor : **Tatano, Toshio**
**No. 2297-6, Ooka**
**Numazu-shi Shizuoka-ken (JP)**
Inventor : **Nakashima, Koichi**
**No.1-413, Minamiohi-cho 1-chome**
**Takatsuki-shi Osaka-fu (JP)**

(74) Representative : **Ellis, Edward Lovell et al**
**MEWBURN ELLIS & CO. 2/3 Cursitor Street**
**London EC4A 1BQ (GB)**

EP 0 325 472 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue Saint-Denis, 75001 PARIS

## Description

The present invention relates to a composition for testing for peptidase-like enzymatic activity, in particular of certain pathogenic oral microorganisms related to periodontal diseases, for the diagnosis or prognosis of contraction of such a disease or to assess the value of therapy. It also relates to kits for carrying out the tests, methods of testing and methods of forming the compositions.

Recently, bacteriological researches on periodontal diseases have advanced and, as the results, it has been found that many spirochetes are detected in sites with periodontal diseases and have good correlation with various clinical indices. Further, it has been also found that anaerobic gram negative bacteria are main pathogenic oral microorganisms for periodontal diseases. Among them, Black-pigmented Bacteroides such as Bacteroides gingivalis are particularly noted and many reports about their pathogenicities are present.

Then, attempts are made to detect these pathogenic bacteria in the oral cavity and apply the results to clinical use so that periodontal diseases can be prevented or treated by prognosticating or diagnosing contraction or progress thereof.

However, there are some drawbacks in detection of these pathogenic oral bacteria by a bacteriological method. For example, detection requires highly skilled technique and special equipments such as use of a dark field microscope and handling of anaerobes, and involves complicated operations. Further, it takes long time and requires skill for cultivation and analysis of the result. Therefore, there are still more difficulties in application thereof to clinical practice.

From the immunological point of view, some attempts are made to detect the presence of the pathogenic microorganisms by determining antibody titer in blood which is humoral immunity to the pathogenic microorganisms, or determining lymphocyte blastformation which is cellular immunity. However, preparation of a specimen requires complicated operations and practical application is still difficult.

Under these circumstances, the present inventors have studied intensively to make detection of the pathogenic oral microorganisms for periodontal diseases applicable to clinical practice possible. As the result, the present inventors have found that some spirochetes in the oral cavity have very specific peptidase-like enzymatic activities and Black-pigmented Bacteroides such as B. gingivalis, B. intermedius and the like also have similar activities, which can be detected specifically, readily and promptly by a colour reaction using particular substrates which precisely reflect periodontal disease conditions. EP-A-255 341 describes these developments.

Separately, the present inventors have found that peptidase-like enzyme can be extremely readily and promptly determined using particular compounds which can promote a colour development reaction, and again have filed a patent application (JP-62-286559).

The present inventors have further studied and have found that the compound which can promote such colour development reaction is applicable to the detection of the above pathogenic oral microorganisms for periodontal diseases and the detection can be accomplished more readily and promptly.

A primary problem presently addressed is to provide an improved composition for testing periodontal diseases which diagnoses or prognosticates contraction or progress of such diseases, or diagnoses therapeutic effect by promptly determining peptidase-like enzymatic activity in a specimen.

According to the present invention, there is provided a composition for testing periodontal diseases which diagnoses or prognosticates contraction or progress of said diseases or diagnoses the therapeutic value by promptly determining peptidase-like enzymatic activity in a specimen, said composition comprises a combination of

(a) a compound of the formula:

X - T - Pro - Y [1]

wherein Pro is proline residue; X is hydrogen or an amino protecting group; Y is a residue of a compound which can increase oxidation reaction rate of a chromogen with a oxidase in the presence of oxygen and is attached to the C-terminal of Pro (hereinafter referred to as an enhancer); and T is an amino acid or peptide residue composed of 0 to 4 amino acids or their protected derivatives the C-terminal of which is attached to the N-terminal of Pro,

(b) a compound of the formula:

X' - Z' - Arg - Y' [2]

wherein Arg is arginine residue; X' is hydrogen or an amino protecting group; Y' is an enhancer residue which is attached to the C-terminal of Arg; and Z' is an amino acid or peptide residue composed of 0 to 4 amino acids or their protected derivatives the C-terminal of which is attached to the N-terminal of Arg,

or a mixture of (a) and (b),

(c) a chromogen, and

(d) an oxidase.

The test using the composition of the present invention includes two reaction systems. The first one is a reaction of a peptidase-like enzyme in a specimen with the substrates of the formulas [1] and [2] to liberate the enhancer. The second one is a reaction to oxidize the chromogen by an enzyme which can oxidize the chromogen in the presence of oxygen and in the presence of the free enhancer to generate a pigment. In the first reaction, the enhancer is generated in proportion to the peptidase-like enzymatic activity in the specimen. In the second reaction, the amount of the enhancer is in proportion to the generation rate of the pigment. That is, the chromogen is oxidized by the action of the oxidase and the pigment is generated with times. The generation rate of the pigment is amplified in the presence of the enhancer. The amount of the pigment generated after a certain period of time is proportion to the amount of the enhancer.

Accordingly, by combining two reactions and determining the pigment generated, the amount of the enhancer is determined and, therefore, peptidase activity in the specimen is determined.

When using the present compositions, the amount of the produced pigment can be determined by the reaction of a specimen such as saliva, dental plaque, gingival crevicular fluid or the like with the substrates of the formulas [1] and [2], preferably, under the optimal conditions (pH 5 to 9) followed by the reaction with the chromogen and oxidase, or a reaction of a specimen with the substrates of the formulas [1] and [2] in the presence of the chromogen and oxidase to readily and promptly diagnose or prognosticate contraction or progress of periodontal diseases or diagnose the therapeutic value.

Compounds of the formulas [1] and/or [2] used as the substrate in the present invention may be known, or readily prepared by known peptide synthesis. In the formulas [1] and [2], the amino protecting groups represented by X and X' may be any amino protecting group known in peptide synthesis such as formyl, acetyl, succinyl, t-butoxy-carbonyl, benzoyl, carbobenzoxy, p-toluenesulfonyl.

T group may be any amino acid or peptide residue which is composed of 0 to 4 amino acids or their protected derivatives wherein C-terminal is attached to N-terminal of the proline residue. Preferably the C-terminal amino acid in the group T is glycine, alanine, lysine, phenylalanine or their protected derivatives. The protected derivative includes OH-protected serine, SH-protected cysteine, β- or γ-COOH protected aspartic acid or glutamic acid, for example, protected with benzyl.

Z' group may be any amino acid or peptide residue which is composed of 0 to 4 amino acids or their protected derivatives wherein C-terminal is attached to N-terminal of the arginine residue. Preferably, the C-terminal amino acid in the group Z' is glycine, lysine, arginine, phenylalanine or their protected derivatives. The protected derivative includes OH-protected serine, SH-protected cysteine, β- or γ-COOH blocked aspartic acid or glutamic acid, for example, protected with benzyl.

The enhancer residues of Y and Y' may be any one which can increase oxidation reaction rate of the chromogen. The typical examples of such an enhancer include aniline derivatives of the formula:

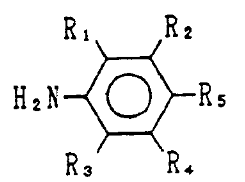

[3]

wherein $R_1$ - $R_4$ are the same or different and are hydrogen, halogen, alkyl having 1 to 4 carbon atoms, sulfone or hydroxyl; and $R_5$ is hydroxyl, amino or amino substituted with alkyl having 1 to 4 carbon atoms, sulfoalkyl having 1 to 4 carbon atoms or hydroxylalkyl having 1 to 4 carbon atoms, or diaminostilben-disulfonic acid such as 3,3'-diaminostilben-4,4'-disulfonic acid (DSDA).

The typical examples of the compound of the formula [3] include, 3,5-dibromo-4-hydroxyaniline (DBHA), 3,5-dichloro-4-hydroxyaniline (DCHA), p-N,N-disulfopropylaminoaniline (SPA), 3,5-diiodo-4-hydroxyaniline (DIHA), p-phenylenediamine (PPD), 4- aminoaniline-3-sulfonic acid DAS), 2-methyl-3,5-dibromo-4-hydroxyaniline (MDBHA), 2,6-dimethyl-3,5-dichloro-4-hydroxyaniline (DMDCHA), 4-N,N-disulfopropylamino-3,5-dibromoaniline (SDBA), 4-(N-ethyl-N-hydroxylethylamino-3,5-dibromoaniline (EHDBA), 4-N,N-diethylamino-3,4-dihydroxyaniline (DEDHA) and the like.

The configuration of each amino acid residue in the compounds of the formulas [1] and [2] is not specifically limited so far as it can be served as the substrate of an peptidase-like enzyme.

As the oxidase used in the present invention, any enzyme which can oxidize the chromogen in the presence of oxygen to produce pigment may be used. For example, bilirubin oxidase (BLOD, ECl, 3, 3, 5), monophenol oxygenase (MPO, ECl, 14, 18, 1), ascorbate oxidase (AOD, ECl, 10, 3, 3), catechol oxidase (ECl, 10, 3, 1), laccase (ECl, 10, 3, 2), o-aminophenol oxidase (ECl, 10, 3, 4), 3-hydroxyanthranilate oxidase (ECl, 10, 3, 5), phenol-2-monooxygenase (ECl, 14, 13, 7) and the like.

As the chromogen, any substance which can develop color by oxidation can be used. To improve sensitivity, those with high molecular extinction coefficient are preferred. However, compounds which show less color development in the absence of the enhancer (corresponding to a blank test), but show significant color development in the presence of the enhance are preferred. Such a chromogen includes, for example, the following compounds:

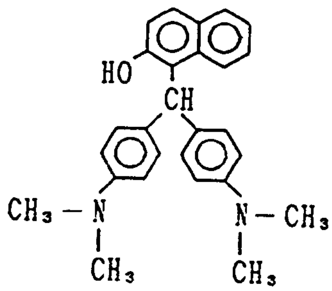

P - 1

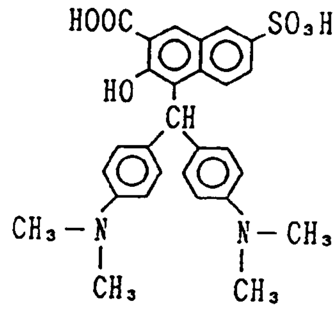

P - 2

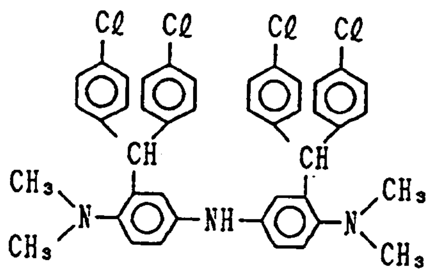

P - 3

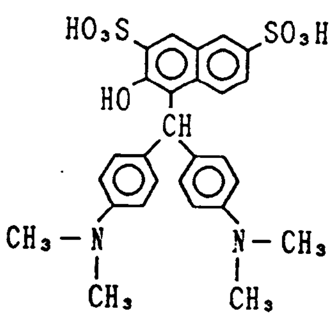

P - 4

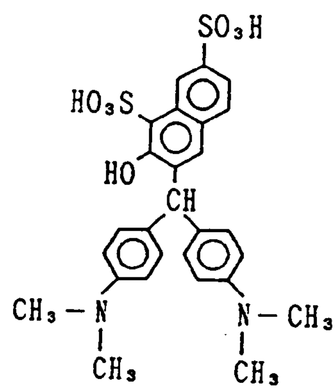

P - 5

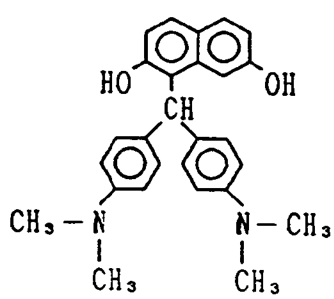

P - 6

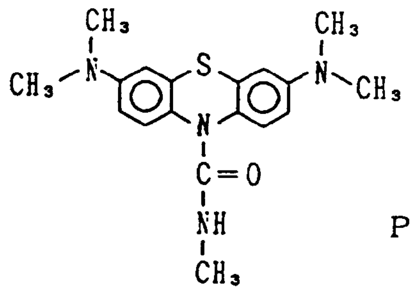

P－7

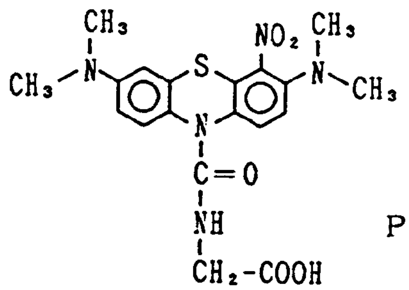

P－8

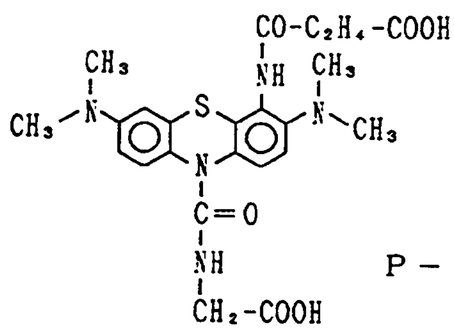

P－9

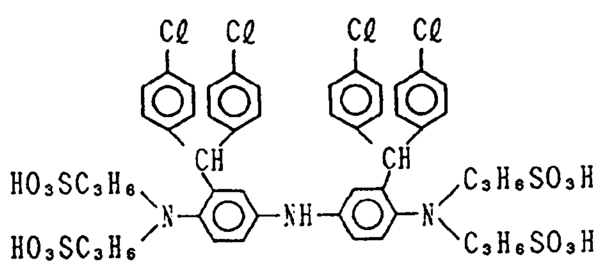

P－10

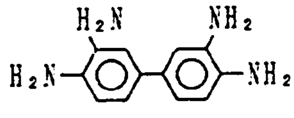

P − 1 1

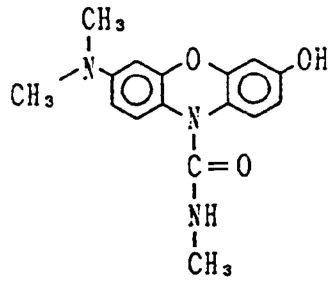

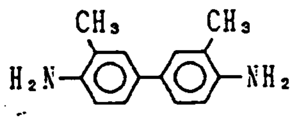

P − 1 2

P − 1 3

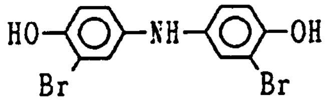

P − 1 4

Maximum absorption of those compounds are shown in the following table.

| Pigment No. | Maximum Absorption (nm) |
|---|---|
| P-1 | 630 |
| 2 | 630 |
| 3 | 755 |
| 4 | 630 |
| 5 | 630 |
| 6 | 630 |
| 7 | 666 |
| 8 | 655 |
| 9 | 668 |
| 10 | 670 |
| 11 | 435 |
| 12 | 650 |
| 13 | 520 |
| 14 | 590 |

The composition of the present invention may be in any preparation form in which the compounds of the formulas [1] and [2] can react as the substrate for the peptidase-like enzyme from the specimen and the liberated enhancer can react to enhance the oxidation condensation reaction of the chromogen with the oxidase.

Generally, e.g. the composition may be a combination of an aqueous solution containing the compound of the formula [1] or [2] or a mixture of the compounds of the formulas [1] and [2] and an aqueous solution containing both oxidase and chromogen, or the compound of the formula [1] or [2] or a mixture thereof and an aqueous solution containing both oxidase and chromogen. Preferably, the composition includes a buffer so that the determination can be carried out at pH of 5 to 9. There can be used any buffer which is usually used, for example, Good buffer, Tris-HCl buffer, phosphate buffer, borate buffer, acetate buffer, veronal buffer, HEPES buffer or the like. The aqueous solution can be prepared according to a conventional method, for example, by dissolving the compounds of the formulas [1] and [2], the chromogen, the oxidase and, if any, the buffer in distilled water. Further, optionally, other additives such as surfactants, preservatives, antibiotics and the like can be added.

In the reaction, preferably, the reagents can be used in the concentration of 0.01 - 10 mg/ml of the chromogen, 0.001 - 1,000 U/ml of the oxidase and 1 mM - 1 M of the buffer, when 10 nM - 10 mM of the compounds of the formulas [1] and [2] are used in the compounding ratio of 1 : 10 - 10 : 1.

The above-mentioned aqueous solutions can be prepared in the form that the solutions contain the compounds of the formulas [1] and [2], the chromogen, the oxidase and, if any, the buffer in the above desired concentrations, and can be directly used for a test. Alternatively, the solutions can be prepared in the form of concentrated solutions which can be optionally diluted with distilled water to give solutions in the desired concentrations before use.

The compositions of the present invention may be solid preparations, for example, dried powder or granules obtained from the above-mentioned aqueous solution according to a known method, a powder mixture obtained by mixing powder ingredients, granules obtained from such a powder mixture, or liquid preparations soaked in carriers such as filter paper, paper disc, sheet, film, stick, sponge, polymers and the like. Further, it may be a kit.

When a test is carried out embodying the present invention, firstly, a specimen is collected. The specimen can be collected according to a known process. For example, gingival crevicular fluid and saliva can be collected with filter paper, capillary, paper point and the like, and dental plaque can be collected with a swab, curette, scaler and the like.

Then, usually the present composition is contacted with the specimen, for example, in a test tube, microtiter plate, cell, vial, plastic cuvette or the like to conduct the reaction, preferably, at pH of 5 to 9. This reaction is usually conducted at 20 to 45°C. The reaction time varies depending on a particular specimen and reaction temperature. Preferably, the reaction is carried out for 5 minutes to 24 hours.

After completion of the reaction, the presence of color development or its intensity is evaluated with the naked eye or with a spectrophotometer to determine the presence or intensity of peptidase-like enzymatic activity in the specimen. Thus, contraction or progress of periodontal diseases can be diagnosed or prognosticated, or therapeutic effect can be diagnosed.

The following Experiments and Examples further illustrate specific embodiments of the invention in detail

Experiment 1

Peptidase-like enzymatic activity of the various kinds of oral anaerobes

Oral anaerobes, viz. four strains of Treponema denticola, five strains of B. gingivalis, four strains of Actinobacillus actinomycetemcomitans, two strains of Actinomyces israelii and three strains of Fusobacterium nucleatum were tested for hydrolytic activity to the substrate of peptidase-like enzyme as follows.

The strains of Treponema were anaerobically cultivated using TYGVS media at 37°C for 7 days, while other bacterial strains were cultivated using brain heart infusion broth at 37°C for 48 to 72 hours. Each culture was diluted to obtain a bacterial cell suspension, the absorption at 660 nm of which was 0.5.

Substrate compounds for peptidase-like enzyme having DBHA as an enhancer were dissolved in 0.1 M Tris-HCl buffer (pH 7.5) at the concentration of 0.2 mM to obtain substrate solutions.

Separately, 0.1 M DIPSO buffer (pH 7.5) containing 5 mg/ml Dispanol®M-32A was mixed with a compound P-2 and AOD to obtain the concentration of 0.2 mg/ml and 50 U/ml, and the resultant was used as a reagent A.

To the substrate solution (1.0 ml) were added the reagent A (1.5 ml) and the cell suspension (0.2 ml) and reacted at 37°C for 30 minutes. After completion of the reaction, the absorption at 630 nm by the produced pigment was determined with a spectrophotometer.

On the basis of the average amount of $\Delta OD_{630}$, the peptidase-like enzymatic activity of each cell suspension was expressed as follows:

- : $\Delta OD_{630} < 0.05$

± : $0.05 < \Delta OD_{630} < 0.1$
+ : $0.1 < \Delta OD_{630} < 0.5$
++ : $0.5 < \Delta OD_{630} < 1.0$
+++ : $1.0 < \Delta OD_{630} < 2.0$
++++ : $2.0 \leqq \Delta OD_{630}$

The results are shown in Table 1. Abbreviations used in Table 1 means the following substrate compounds.

GR: glycyl-arginine-DBHA
Bz-GR: N-benzoyl-glycyl-arginine-DBHA
Z-VGR: N-carbobenzoxy-valyl-glycyl-arginine-DBHA
GP: glycyl-proline-DBHA
Z-KP: N-carbobenzoxy-lysyl-proline-DBHA
Bz-RGFP: N-benzoyl-arginyl-glycyl-phenylalanyl-proline-DBHA

Table 1

| Strain | Substrate | | | | |
|---|---|---|---|---|---|
| | GR | Bz-GR | Z-VGR | GP | Z-KP |
| Treponema denticola | + | ++ | +++ | ++ | + |
| Bacteroides gingivalis | ++ | +++ | +++ | +++ | ++ |
| Actinobacillus actinomycetemcomitans | - | - | - | - | - |
| Actinomyces israelii | - | - | - | - | - |
| Fusobacterium nucleatum | - | - | - | - | - |

Table 1 (continued)

| Strain | Substrate | | | | |
|---|---|---|---|---|---|
| | Bz-RGFP | GR + GP | Bz-GR + Z-KP | Z-VGR + Bz-RGFP | Bz-GR + Bz-RGFP |
| Treponema denticola | + | +++ | +++ | ++++ | +++ |
| Bacteroides gingivalis | ++ | +++ | ++++ | ++++ | ++++ |
| Actinobacillus actinomycetemcomitans | - | - | - | - | - |
| Actinomyces israelli | - | - | - | - | - |
| Fusobacterium nucleatum | - | - | - | - | - |

As is shown in Table 1, among the oral anaerobes, pathogenic spirochetes for periodontal diseases (Treponema denticola) and B. gingivalis show specific peptidase-like enzymatic activity. When the substrate having Arg at the C-terminal thereof is used together with the substrate having Pro at the C-terminal thereof, the enzymatic activity is increased twice or three times as much as that obtained by using either substrate alone.

Experiment 2

Comparison of sensitivity of the present method and that of a conventional method

Peptidase-like enzymatic activities of Treponema denticola ATCC35405 and B. gingivalis ATCC33277 were determined according to one method of the present invention and a conventional method, and sensitivities of the two methods were compared.

Cultivation of the the bacteria was conducted according to the same manner as described in Experiment 1. The culture medium was diluted to prepare bacterial cell suspensions, the absorptions of which at 660 nm were 0.5 and 0.1.

As substrates, N-benzoyl-arginyl-glycyl-phenylalanyl-proline-DBHA (Bz-Arg-Gly-Phe-Pro-DBHA) and N-carbobenzoxy-glycyl-glycyl-arginine-DBHA (Z-Gly-Gly-Arg-DBHA) were used and dissolved in 0.1 M phosphate buffer (pH 7.0) at the concentration of 0.2 mM to prepare the substrate solution.

The determination of enzymatic activity embodying the present invention was conducted according to the same manner as described in Experiment 1, wherein P-1 was used instead of P-2 as the chromogen.

Separately, as the conventional method, bacterial cell suspension (0.2 ml) was added to 0.2 mM substrate solution (1.0 ml). After completion of the reaction at 37°C for 30 minutes, 12 mg/ml N-ethyl-N-(3-methylphenyl)-N'-succinyl ethylene diamine (EMSE) (1.0 ml) and 0.5 mg/ml potassium ferricyanide (0.5 ml) were added. After reaction at 37°C for 5 minutes, the change of absorbance by produced pigment at 710 nm was determined by a spectrophotometer.

The results are shown in Table 2. The data were OD values determined by the conventional method and the method embodying the invention.

As is obvious from the table, any combination of the two substrates and the cell solution, OD value obtained by the present method is 15 to 20 times as much as that obtained by the conventional method. Further, those undetectable by the conventional method due to a small amount of bacteria (enzyme) can be detected by the present method.

Table 2

| Strain | Amount of Bacteria $OD_{660}$ | Bz-Arg-Gly-Phe-Pro-DBHA Conventional Method $OD_{710}$ | Bz-Arg-Gly-Phe-Pro-DBHA Present Method $OD_{630}$ | Z-Gly-Gly-Arg-DBHA Conventional Method $OD_{710}$ | Z-Gly-Gly-Arg-DBHA Present Method $OD_{630}$ |
|---|---|---|---|---|---|
| B. gingivalis | 0.1 | 0.01 | 0.02 | 0.08 | 2.25 |
| | 0.5 | 0.13 | 2.52 | 0.41 | 8.84 |
| Treponema denticola | 0.1 | 0 | 0.08 | 0.01 | 0.24 |
| | 0.5 | 0.03 | 0.44 | 0.06 | 1.32 |

Experiment 3

Sensitivities of methods embodying the invention were compared with the conventional method in the same way as in Experiment 2, except that:

the cell solution of B.gingivalis prepared in Experiment 2 and having absorbance of 0.5 at 660nm was used; compound P-2 was used as the chromogen, and various oxidases were used instead of AOD.

Sensitivity was expressed as a ratio of $OD_{630}$ (embodiment of invention) to $OD_{710}$ (conventional method).

The results are shown in Table 3. When various oxidases were used instead of AOD, higher sensitivity compared to the conventional method was obtained.

Table 3

| Oxidase | Concentration of Oxidase (U/ml) | Ratio of Sensitivity |
|---|---|---|
| MPO | 0.2 | 16.5 |
| CAO | 1.2 | 78.1 |
| Laccase | 0.05 | 4.3 |
| APO | 0.02 | 6.9 |
| HAO | 2.5 | 9.2 |
| PMO | 0.08 | 11.6 |
| BLOD | 0.02 | 5.2 |

Experiment 4

According to the same manner as described in Experiment 3, sensitivities of various methods embodying the invention were compared to that of the conventional method except that AOD was used as the oxidase, and, as the enhancer, SPA, DIHA, DSDA, PPD, DAS, MDBHA, DMDCHA, SDBA, EHDBA and DEDHA were used instead of DBHA.

As the result, the ratio of the sensitivity shown in Table 4 was obtained.

Table 4

| Enhancer | Ratio of Sensitivity |
|---|---|
| SPA | 5.2 |
| DIHA | 46.1 |
| DSDA | 3.4 |
| PPD | 6.8 |
| DAS | 4.4 |
| MDBHA | 16.6 |
| DMDCHA | 20.8 |
| SDBA | 5.0 |
| EHDBA | 11.4 |
| DEDHA | 8.9 |

Experiment 5

Correlation with clinical state (1)

Specimens of gingival crevicular fluid were collected with paper points from five subjects who were considered to be healthy based on their clinical state, six subjects with gingivalis and six subjects with periodontitis. Each specimen was dispersed in Ringer's solution (1.5 ml), and the relative amount of spirochete to total bacteria was determined with a phase contrast microscope according to the following equation:

$$\text{Relative amount} = \frac{\text{Number of spirochetes in 1ml of Ringer's solution}}{\text{Number of all bacteria in 1 ml of Ringer's solution}} \times 100\ (\%)$$

Further, the Ringer's solution (0.2 ml) was tested for hydrolytic activity using the substrate solution prepared in the same manner as described in Experiment 1. As the substrate, there were used compounds of the formula [1], viz. glycyl-proline-DBHA (GP) and N-benzoyl-arginyl-glycyl-phenylalanyl-proline-DBHA (Bz-RGFP) and compounds of the formula [2], viz. N-carbobenzoxy-valyl-glycyl-arginine-DBHA (Z-VGR), N-benzoyl-glycyl-arginine-DBHA (Bz-GR) were used alone or in combination thereof.

The results are shown in Table 5. In Table 5, enzymatic activity was expressed in the same manner as in Experiment 1.

As shown in Table 5, enzymatic activity has correlation with the amount of spirochete and the clinical state. When the substrates are used in combination thereof, the activity is increased compared with that obtained by using the substrate alone.

Table 5

| Subject | Amount of Spirochete (%) | Z-VGR | Bz-GR | GP | Substrate Bz-RGFP | Z-VGR + GP | Bz-GR + B-RGFP |
|---|---|---|---|---|---|---|---|
| Healthy | | | | | | | |
| 1 | 0.3 | - | - | - | - | - | - |
| 2 | 1.4 | + | ± | ± | + | + | + |
| 3 | 0.5 | - | - | - | - | - | - |
| 4 | 0.2 | - | - | - | - | - | - |
| 5 | 1.2 | - | - | ± | - | ± | ± |
| Gingivitis | | | | | | | |
| 1 | 5.4 | + | + | + | + | + | + |
| 2 | 10.3 | + | + | + | + | ++ | ++ |
| 3 | 18.7 | ++ | + | ++ | ++ | ++ | ++ |
| 4 | 3.4 | - | - | - | - | - | - |
| 5 | 6.9 | - | ± | ± | - | ± | ± |
| 6 | 9.4 | + | ± | + | + | + | ++ |
| Periodontitis | | | | | | | |
| 1 | 20.5 | +++ | ++ | +++ | +++ | ++++ | ++++ |
| 2 | 35.4 | +++ | ++ | +++ | +++ | ++++ | ++++ |
| 3 | 29.3 | +++ | ++ | +++ | +++ | ++++ | ++++ |
| 4 | 25.3 | +++ | ++ | ++ | +++ | ++++ | ++++ |
| 5 | 40.5 | +++ | ++ | +++ | +++ | ++++ | ++++ |
| 6 | 39.7 | +++ | ++ | +++ | +++ | ++++ | ++++ |

Experiment 6

Correlation with clinical state (2)

A saliva mixture collected from a group of ten healthy subjects, a group of ten subjects with adult periodontitis or a group of four subjects with localized juvenile periodontitis was centrifuged to obtain a supernatant. The supernatant was used as a specimen and peptidase-like enzymatic activity obtained in accordance with the present invention was compared with that obtained by the conventional method according to the same manner as described in Experiment 2. In this experiment, the reaction was conducted for 15 minutes. As the substrates, there were used N-carbobenzoxy-glycyl-arginine-DIHA (Z-GR), N-benzoyl-arginyl-glycyl-phenylalanyl-proline-DIHA (Bz-RGFP), alone or in combination thereof.

The results are shown in Table 6. As in Experiment 2, the results are expressed as $OD_{630}$ and $OD_{710}$.

As shown in Table 6, both groups of subjects with adult periodontitis and localized juvenile periodontitis have higher activity than that of healthy subjects in both methods, and difference is statistically significant. Further, in the method embodying the invention, OD value is ten times as high as that of the conventional

method and there is significant difference in OD values between the diseased groups and the healthy group. Particularly, using the substrate having arginine at the C-terminal together with the substrate having proline at the C-terminal, the diseased group showed more than about 1.5 times higher activity. Therefore, by determination of enzymatic activity according to a method embodying the invention, periodontal diseases may be promptly diagnosed or prognosticated and therapeutic effect can be objectively evaluated.

Table 6

| Method | Substrate | Group: Healthy subjects | Adult Periodontitis subjects | Localized juvenile periodontitis subjects |
|---|---|---|---|---|
| Conventional Method ($OD_{710}$) | Z-GR | 0 ± 0 | 0.1 ± 0.1** | 0.1 ± 0.1** |
| | Bz-RGFP | 0 ± 0 | 0.1 ± 0.1** | 0.1 ± 0.1** |
| | Z-GR + B-RGFP | 0 ± 0 | 0.2 ± 0.1** | 0.1 ± 0.1** |
| Present Method ($OD_{630}$) | Z-GR | 0.1 ± 0.1 | 1.4 ± 0.6* | 1.0 ± 0.3** |
| | Bz-RGFP | 0.1 ± 0.1 | 1.2 ± 0.5* | 0.8 ± 0.3* |
| | Z-GR + B-RGFP | 0.2 ± 0.1 | 1.9 ± 0.6* | 1.5 ± 0.6* |

*: $P < 0.01$
**: $P < 0.05$

## Example 1

As the substrate solution, a solution of N-carbobenzoxy-valyl-glycyl-arginine-DBHA in distilled water (2 mM) was prepared. 0.1 M Tris-HCl buffer (pH 7.0) was prepared and used as buffer A. 0.1 M DIPSO buffer (pH 7.5) containing Dispanol® M-32A (5 mg/ml) was used as buffer B. AOD was added to buffer B to 200 U/ml and was used as the oxidase solution. The compound P-3 was dissolved in buffer B to 0.4 mg/ml and was used as the chromogen solution.

These reagents were combined and used as a kit for testing periodontal diseases.

This kit can be used for diagnosis or prognostication of periodontal diseases as follows.

A paper point is inserted into the gingival crevice of a subject for 30 seconds to collect a specimen. The substrate solution (0.1 ml) and the buffer A (0.9 ml) are mixed and the specimen is added to the mixture. The mixture is allowed to react at 37°C for 30 minutes. To the resultant are further added the oxidase solution (0.5 ml) and the chromogen solution (0.5 ml), and the mixture is allowed to react at 37°C for 30 minutes. After completion of the reaction, a color tone is observed by the naked eye. The intensity of blue color is evaluated by comparing with a blank control. Heavy blue color shows that the subject has periodontal diseases.

Example 2

The substrate reagent was prepared by soaking a paper disc (diameter : 0.6 cm) with N-carbobenzoyl-glycycl-glycyl-arginine-SPA (500 nmol) and N-benzoyl-prolyl-alanyl-glycyl-proline-SPA (500 nmol). 0.1 M phosphate buffer (pH 7.0) containing Triton X-100 at 5 mg/ml was used as buffer C. A paper disc (diameter : 0.6 cm) soaked with BLOD (0.02U) and the compound P-4 (1 mg) was used as the color producing reagent. These reagents were combined and used as a kit for testing periodontal diseases.

This kit can be used for diagnosis or prognostication of periodontal diseases as follows.

The buffer solution C (400 µl) is placed in a vial to which are added saliva mixture (100 µl) collected from a subject and the paper disc of the substrate reagent. The mixture is allowed to react at 37°C for 15 minutes. Further, a paper disc of the color producing reagent is added and allowed to react at 37°C for 15 minutes. After completion of the reaction, the color development is evaluated by the naked eye according to the same manner as described in Example 1.

Example 3

N-Carbobenzoyl-arginine-DIHA (500 nmol), N-benzoyl-alanyl-proline-DIHA (250 nmol), HAO (2.5 U) and the compound P-5 were mixed and lyophilized in an ampoule (inner diameter : 5 mm; length ; 3 cm) to prepare the reaction reagent.

0.1 M DIPSO buffer (pH 7.5) containing Triton X-100 (5 mg/ml) was prepared and used as a buffer.

These reagents were combined and used as a kit for testing periodontal diseases.

This kit can be used for diagnosis or prognostication of periodontal diseases as follows.

A paper strip is inserted into the gingival crevice of a subject for 30 seconds to collect a specimen, The buffer (1 ml) is poured in an ampoule containing reagent to dissolve it. The specimen is added to the solution and reacted at 37°C for 30 minutes. After completion of the reaction, the color development is evaluated with the naked eye according to the same manner as described in Example 1.

Example 4

N-t-Butoxycarbonyl-valyl-leucyl-glycyl-arginine-DBHA, MPO, the compound P-8 (5 mg/ml) were dissolved in 0/1 M-DIPSO buffer (pH 7.5) containing Dispanol®M32A at concentrations of 200 nmol/ml, 0.2 U/ml, 0.2 mg/ml, respectively. A circular filter paper (diameter : 1 cm) was soaked with the solution (100 µl), dried to prepare a test paper.

This test paper can be used for diagnosis or prognostication of periodontal diseases as follows.

Saliva mixture (100 µl) collected from a subject is added dropwise to the test paper and reacted at room temperature for 20 minutes. After completion of the reaction, the color tone of the test paper is evaluated with the naked eye. Comparing to the control sample which is prepared by adding water instead of the specimen, the intensity of blue color is evaluated. Strong blue shows that the subjects have periodontal diseases.

Example 5

AOD (3000 U) was lyophilized in a vial (shell diameter : 30 mm; length : 60 mm) and used as reagent A1 (for 30 specimens).

N-Carbobenzoxy-glycyl-glycyl-arginine-DBHA (50 nmol) and the compound P-7 (0.5 mg) were mixed and lyophilized in a vial (shell diameter : 18 mm; length : 33 mm) and used as reagent A2 (for one specimen).

0.1 M PIPES buffer (pH 7.0) containing 1 mg/ml dispanol (30 ml) was charged in a pet bottle (75 ml volume) and used as reagent B (for 30 specimens).

An aqueous solution containing sodium diethyldithiocarbamate (5 mg/ml) and sodium nitride (2 mg/ml) (pH 9.5) was charged in an eye drop bottle (50 ml volume) and used as a reaction terminator solution.

These reagents were combined and used as kit for testing periodontal diseases.

This kit can be used for diagnosis or prognostication of periodontal diseases as follows.

A bottle of reagent A1 is dissolved with a bottle of reagent B to prepare AOD solution. A paper point is inserted into the gingival crevice of a subject for 30 seconds to collect a specimen and poured in a vial containing reagent A2. To the mixture is added AOD solution (1 ml). After 15 minutes, the reaction terminator solution (one drop) is added and the mixture is stirred to stop the reaction. After addition of the reaction terminator solution, color development is observed with the naked eye within 4 hours in the same manner as described in Example 1.

## Claims

1. A composition or kit for testing for periodontal diseases by determining peptidase-like enzymatic activity in a specimen, which composition or kit comprises a combination of

   A.

   (i) a compound of the formula:

   X - T - Pro - Y [1]

   wherein Pro is proline residue; X is hydrogen or an amino protecting group; Y is a residue of a compound (hereinafter "enhancer") capable of increasing the oxidation rate of a chromogen with an oxidase in the presence of oxygen, attached to the C-terminal of Pro; and T is an amino acid or peptide residue composed of 0 to 4 amino acids or a protected derivative thereof, the C-terminal of which is attached to the N-terminal of Pro, or

   (ii) a compound of the formula:

   X' - Z' - Arg - Y' [2]

   wherein Arg is arginine residue; X' is hydrogen or an amino protecting group; Y' is an enhancer residue which is attached to the C-terminal of Arg; and Z' is an amino acid or peptide residue composed of 0 to 4 amino acids or their protected derivatives, the C-terminal of which is attached to the N-terminal of Arg, or

   (iii) a mixture of (i) and (ii),

   B. a chromogen, and

   C. an oxidase.

2. A composition according to claim 1, wherein the enhancer is an aniline derivative of the formula:

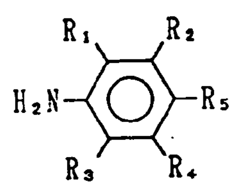

[3]

   wherein $R_1$ - $R_4$ are the same or different and are hydrogen, halogen, alkyl having 1 to 4 carbon atoms, sulfone or hydroxyl; and R is hydroxyl, amino or amino substituted with alkyl having 1 to 4 carbon atoms sulfoalkyl having 1 to 4 carbon atoms or hydroxylalkyl having 1 to 4 carbon atoms, or diaminostilbenedisulfonic acid.

3. A composition according to claim 1 or claim 2, wherein the C-terminal amino acid residue in the group T is a residue of glycine, alanine, lysine, phenylalanine or a protected derivative thereof.

4. A composition according to any of claims 1, 2 or 3, wherein the C-terminal amino acid residue in the group Z is a residue of glycine, lysine, arginine, phenylalanine or a protected derivative thereof.

5. A method of testing for peptidase-like enzymatic activity of a specimen, comprising reacting the specimen with:

   A.

   (i) a compound of the formula:

   X - T - Pro - Y [1]

   wherein Pro is proline residue; X is hydrogen or an amino protecting group; Y is a residue of a compound (hereinafter "enhancer") capable of increasing the oxidation rate of a chromogen with an oxidase in the presence of oxygen, attached to the C-terminal of Pro; and T is an amino acid or peptide residue composed of 0 to 4 amino acids or a protected derivative thereof, the C-terminal of which is attached to the N-terminal of Pro or

(ii) a compound of the formula:

X' - Z' - Arg - Y' [2]

wherein Arg is arginine residue X' is hydrogen or an amino protecting group; Y' is an enhancer residue which is attached to the C-terminal of Arg; and Z' is an amino acid or peptide residue composed of 0 to 4 amino acids or their protected derivatives, the C-terminal of which is attached to the N-terminal of Arg, or

(iii) a mixture of (i) and (ii),

whereby any such enzymatic activity liberates enhancer, and providing in the system

B. a chromogen, and

C. an oxidase

whereby the degree of enhanced oxidation of chromogen by the oxidase indicates by colour the degree of said activity.

6. A method according to claim 5, wherein reactants B and C are introduced subsequent to reaction of A with the specimen, or wherein all three of A, C and B are provided mixed together for reaction, optionally in a common carrier, solution or dispersion.

7. A method of diagnosis or prognosis of periodontal disease comprising a method according to claim 5 or claim 6 carried out on a specimen permanently removed from the human or animal body.

8. A method of preparing a composition for testing for peptidase-like enzymatic activity, comprising combining:

A.

(i) a compound of the formula:

X - T - Pro - Y [1]

wherein Pro is proline residue; X is hydrogen or an amino protecting group; Y is a residue of a compound (hereinafter "enhancer") capable of increasing the oxidation rate of a chromogen with an oxidase in the presence of oxygen, attached to the C-terminal of Pro; and T is an amino acid or peptide residue composed of 0 to 4 amino acids or a protected derivative thereof, the C-terminal of which is attached to the N-terminal of Pro, or

(ii) a compound of the formula:

X' - Z' - Arg - Y' [2]

wherein Arg is arginine residue X' is hydrogen or an amino protecting group; Y' is an enhancer residue which is attached to the C-terminal of Arg; and Z' is an amino acid or peptide residue composed of 0 to 4 amino acids or their protected derivatives, the C-terminal of which is attached to the N-terminal of Arg, or

(iii) a mixture of (i) and (ii),

B. a chromogen, and

C. an oxidase.

9. A method according to claim 8 comprising mixing together components A, B and C, optionally in a common carrier, solution or dispersion.

## Patentansprüche

1. Zusammensetzung oder Kit für die Untersuchung paradontaler Krankheiten durch Bestimmung der Peptidase-artigen enzymatischen Aktivität in einer Probe, dadurch **gekennzeichnet**, daß die Zusammensetzung oder das Kit eine Kombination aus

A.

(i) einer Verbindung der Formel:

X - T - Pro - Y [1]

worin Pro den Prolinrest bedeutet; X Wasserstoff oder eine Aminoschutzgruppe bedeutet; Y ein Rest einer Verbindung (im folgenden als "Enhancer") bezeichnet, der die Oxidationsgeschwindigkeit eines Chromogens mit einer Oxidase in Anwesenheit von Sauerstoff erhöhen kann und an das C-Terminal von Pro gebunden ist, bedeutet; und T einen Aminosäure- oder Peptidrest, der aus 0 bis 4 Aminosäuren besteht, oder ein geschütztes Derivat davon, dessen C-Terminal an das N-Terminal von Pro gebunden ist, bedeutet, oder

(ii) einer Verbindung der Formel:

X' - Z' - Arg - Y' [2]

worin Arg den Argininrest bedeutet; X' Wasserstoff oder eine Aminoschutzgruppe bedeutet; Y' einen Enhancer-Rest, der an das C-Terminal von Arg gebunden ist, bedeutet; und Z' einen Aminosäure- oder Peptidrest, der aus 0 bis 4 Aminosäuren besteht, oder geschützte Derivate davon, wobei das C-Terminal davon an das N-Terminal von Arg gebunden ist, bedeutet, oder

(iii) einem Gemisch aus (i) und (ii),

B. einem Chromogen, und

C. einer Oxidase

enthält.

2. Zusammensetzung nach Anspruch 1, dadurch **gekennzeichnet**, daß der Enhancer ein Ainilinderivat der Formel:

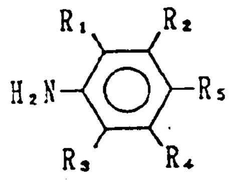

[3]

worin $R_1$ - $R_4$ gleich oder unterschiedlich sind und Wasserstoff, Halogen, Alkyl mit 1 bis 4 Kohlenstoffatomen, Sulfon oder Hydroxyl bedeuten; und $R_5$ Hydroxyl, Amino oder Amino, substituiert mit Alkyl, mit 1 bis 4 Kohlenstoffatomen, Sulfoalkyl mit 1 bis 4 Kohlenstoffatomen oder Hydroxyalkyl mit 1 bis 4 Kohlenstoffatomen, oder Diaminostilbendisulfonsäure ist.

3. Zusammensetzung nach Anspruch 1 oder 2, dadurch **gekennzeichnet**, daß der C-terminale Aminosäurerest in der Gruppe T ein Rest von Glycin, Alanin, Lysin, Phenylalanin oder einem geschützten Derivat davon ist.

4. Zusammensetzung nach einem der Ansprüche 1, 2 oder 3, dadurch **gekennzeichnet**, daß der C-terminale Aminosäurerest in der Gruppe Z ein Rest von Glycin, Lysin, Arginin, Phenylalanin oder einem geschützten Derivat davon ist.

5. Verfahren für die Untersuchung der Peptidase-artigen enzymatischen Aktivität einer Probe, dadurch **gekennzeichnet**, daß die Probe umgesetzt wird mit

A.

(i) einer Verbindung der Formel:

X - T - Pro - Y [1]

worin Pro den Prolinrest bedeutet; X Wasserstoff oder eine Aminoschutzgruppe bedeutet; Y ein Rest einer Verbindung (im folgenden als "Enhancer") bezeichnet, der die Oxidationsgeschwindigkeit eines Chromogens mit einer Oxidase in Anwesenheit von Sauerstoff erhöhen kann und an das C-Terminal von Pro gebunden ist, bedeutet; und T einen Aminosäure- oder Peptidrest, der aus 0 bis 4 Aminosäuren besteht, oder ein geschütztes Derivat davon, dessen C-Terminal an das N-Terminal von Pro gebunden ist, bedeutet, oder

(ii) einer Verbindung der Formel:

X' - Z' - Arg - Y' [2]

worin Arg den Argininrest bedeutet; X' Wasserstoff oder eine Aminoschutzgruppe bedeutet; Y' einen Enhancer-Rest, der an das C-Terminal von Arg gebunden ist, bedeutet; und Z' einen Aminosäure- oder Peptidrest, der aus 0 bis 4 Aminosäuren besteht, oder geschützte Derivate davon, wobei das C-Terminal davon an das N-Terminal von Arg gebunden ist, bedeutet, oder

(iii) einem Gemisch aus (i) und (ii),

wobei irgendeine solche enzymatische Aktivität den Enhancer freisetzt, und in dem System

B. ein Chromogen, und

C. eine Oxidase

vorgesehen sind, wobei der Grad der verstärkten Oxidation des Chromogens durch die Oxidase durch Verfärbung den genannten Aktivitätsgrad anzeigt.

6. Verfahren nach Anspruch 5, dadurch **gekennzeichnet**, daß die Reaktionsteilnehmer B und C anschließend an die Reaktion von A mit der Probe zugegeben werden, oder daß alle drei, nämlich A, C und B, zusammen vermischt für die Reaktion, gegebenenfalls in einem gemeinsamen Träger, in Lösung oder Dispersion, vorliegen.

7. Verfahren für die Diagnose oder Prognose paradontaler Krankheiten, dadurch **gekennzeichnet**, daß ein Verfahren nach Anspruch 5 oder Anspruch 6 mit einer Probe durchgeführt wird, die permanent aus dem Menschen- oder Tierkörper entfernt wurde.

8. Verfahren zur Herstellung einer Zusammensetzung für die Untersuchung der Peptidase-artigen enzymatischen Aktivität, dadurch **gekennzeichnet**, daß:

A.

(i) eine Verbindung der Formel:

X - T - Pro - Y [1]

worin Pro den Prolinrest bedeutet; X Wasserstoff oder eine Aminoschutzgruppe bedeutet; Y ein Rest einer Verbindung (im folgenden als "Enhancer") bezeichnet, der die Oxidationsgeschwindigkeit eines Chromogens mit einer Oxidase in Anwesenheit von Sauerstoff erhöhen kann und an das C-Terminal von Pro gebunden ist, bedeutet; und T einen Aminosäure- oder Peptidrest, der aus 0 bis 4 Aminosäuren besteht, oder ein geschütztes Derivat davon, dessen C-Terminal an das N-Terminal von Pro gebunden ist, bedeutet, oder

(ii) eine Verbindung der Formel:

X' - Z' - Arg - Y' [2]

worin Arg den Argininrest bedeutet; X' Wasserstoff oder eine Aminoschutzgruppe bedeutet; Y' einen Enhancer-Rest, der an das C-Terminal von Arg gebunden ist, bedeutet; und Z' einen Aminosäure- oder Peptidrest, der aus 0 bis 4 Aminosäuren besteht, oder geschützte Derivate davon, wobei das C-Terminal davon an das N-Terminal von Arg gebunden ist, bedeutet, oder

(iii) ein Gemisch aus (i) und (ii),

B. ein Chromogen, und

C. eine Oxidase

vermischt werden.

9. Verfahren nach Anspruch 8, dadurch **gekennzeichnet**, daß die Komponenten A, B und C gegebenenfalls in einem gemeinsamen Träger, in Lösung oder Dispersion, vermischt werden.

## Revendications

1. Composition ou trousse pour déceler les paradontolyses par détermination d'une activité enzymatique analogue à celle de la peptidase dans un échantillon, laquelle composition ou trousse comprend une combinaison de :

A.

(i) un composé de formule :

X - T - Pro - Y [1]

dans laquelle Pro est un résidu de proline, X est l'hydrogène ou un groupe protecteur de groupe amine, Y est un résidu d'un composé (dans la suite "stimulateur") capable d'augmenter le taux d'oxydation d'un chromogène par une oxydase en présence d'oxygène, fixé à l'extrémité C de Pro, et T est un résidu d'acide aminé ou de peptide constitué par 0 à 4 acides aminés ou par un dérivé protégé de ceux-ci, dont l'extrémité C est liée a l'extrémité N de Pro, ou

(ii) un composé de formule :

X' - Z' - Arg - Y' [2]

dans laquelle Arg est un résidu d'arginine, X' est l'hydrogène ou un groupe protecteur de groupe amine, Y' est un résidu de stimulateur qui est lié à l'extrémité C de Arg, et Z' est un résidu d'acide aminé ou de peptide constitué par 0 à 4 acides aminés ou par leurs dérivés protégés, dont l'extrémité C est liée a l'extrémité N de Arg, ou

(iii) un mélange de (i) et de (ii),

B. Un chromogène, et

C. Une oxydase.

**2.** Composition selon la revendication 1, dans laquelle le stimulateur est un dérivé de l'aniline de formule:

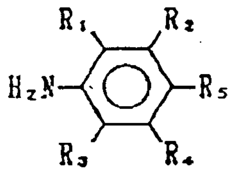

[3]

dans laquelle $R_1$ à $R_4$ sont identiques ou différents et sont des atome d'hydrogène, d'halogène, des groupes alkyle de 1 à 4 atomes de carbone, sulfone ou hydroxyle, et $R_5$ est un groupe hydroxyle, amine ou amine substitué par un groupe alkyle de 1 à 4 atomes de carbone, sulfoalkyle de 1 à 4 atomes de carbone ou hydroxyalkyle de 1 à 4 atomes de carbone, ou acide diaminostilbènedisulfonique.

**3.** Composition selon la revendication 1 ou la revendication 2, dans laquelle le résidu d'acide aminé C-terminal du groupe T est un résidu de glycine, d'alanine, de lysine, de phénylalanine ou un dérivé protégé de celui-ci.

**4.** Composition selon l'une quelconque des revendications 1, 2 ou 3, dans laquelle le résidu d'acide aminé C-terminal du groupe Z est un résidu de glycine, de lysine, d'arginine, de phénylalanine ou un dérivé protégé de celui-ci.

**5.** Procédé pour déceler une activité enzymatique analogue à celle de la peptidase d'un échantillon, comprenant la réaction de l'échantillon avec :

A.

(i) un composé de formule :

X - T - Pro - Y [1]

dans laquelle Pro est un résidu de proline, X est l'hydrogène ou un groupe protecteur de groupe amine, Y est un résidu d'un composé (dans la suite "stimulateur") capable d'augmenter le taux d'oxydation d'un chromogène par une oxydase en présence d'oxygène, fixé à l'extrémité C de Pro, et T est un résidu d'acide aminé ou de peptide constitué par 0 à 4 acides aminés ou par un dérivé protégé de ceux-ci, dont l'extrémité C est liée à l'extrémité N de Pro, ou

(ii) un composé de formule :

X' - Z' - Arg - Y' [2]

dans laquelle Arg est un résidu d'arginine, X' est l'hydrogène ou un groupe protecteur de groupe aminé, Y' est un résidu de stimulateur qui est lié à l'extrémité C de Arg, et Z' est un résidu d'acide aminé ou de peptide constitué par 0 à 4 acides aminés ou par leurs dérivés protégés, dont l'extrémité C est liée à l'extrémité N de Arg, ou

(iii) un mélange de (i) et de (ii),

toute activité enzymatique de ce type libérant le stimulateur, et la fourniture dans le système :

B. D'un chromogène, et

C. D'une oxydase,

dans lequel le degré de l'oxydation augmentée du chromogène par l'oxydase indique par une couleur le degré de ladite activité.

**6.** Procédé selon la revendication 5, dans lequel les corps réagissants B et C sont introduits à la suite de la réaction de A avec l'échantillon, ou dans lequel A, C et B sont tous trois fournis en mélange entre eux pour la réaction, éventuellement dans un véhicule, solution ou dispersion commun.

**7.** Procédé de diagnostic ou de pronostic d'un paradontolyse, comprenant un procédé selon la revendication 5 ou la revendication 6 mis en oeuvre sur un échantillon prélevé en permanence sur l'organisme humain ou animal.

**8.** Procédé de préparation d'une composition pour déceler une activité enzymatique analogue à celle de la peptidase, comprenant la combinaison de :

A.

(i) un composé de formule :

X - T - Pro - Y [1]

dans laquelle Pro est un résidu de proline, X est l'hydrogène ou un groupe protecteur de groupe amine, Y est un résidu d'un composé (dans la suite "stimulateur") capable d'augmenter le taux d'oxydation d'un chromogène par une oxydase en présence d'oxygène, fixé à l'extrémité C de Pro, et T est un résidu d'acide aminé ou de peptide constitué par 0 à 4 acides aminés ou par un dérivé protégé de ceux-ci, dont l'extrémité C est liée à l'extrémité N de Pro, ou

(ii) un composé de formule :

X' - Z' - Arg - Y' [2]

dans laquelle Arg est un résidu d'arginine, X' est l'hydrogène ou un groupe protecteur de groupe amine, Y' est un résidu de stimulateur qui est lié à l'extrémité C de Arg, et Z' est un résidu d'acide aminé ou de peptide constitué par 0 à 4 acides aminés ou par leurs dérivés protégés, dont l'extrémité C est liée à l'extrémité N de Arg, ou

(iii) un mélange de (i) et de (ii).

B. Un chromogène, et

C. Une oxydase.

**9.** Procédé selon la revendication 8, comprenant le mélange entre eux des composants A, B et C, éventuellement dans un véhicule, solution ou dispersion commun.